(19) Europäisches Patentamt
European Patent Office
Office européen des brevets

(11) **EP 2 846 135 B1**

(12) **EUROPEAN PATENT SPECIFICATION**

(45) Date of publication and mention
of the grant of the patent:
**29.04.2020   Bulletin 2020/18**

(51) Int Cl.:
*G01D 3/032* *(2006.01)*     *G01B 11/14* *(2006.01)*
*G01N 33/00* *(2006.01)*     *H04M 1/725* *(2006.01)*

(21) Application number: **14003126.1**

(22) Date of filing: **10.09.2014**

(54) **Portable Electronic Device with Environmental Sensor**

Tragbare elektronische Vorrichtung mit Umgebungssensor

Dispositif électronique portable avec capteur environnemental

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2013   CH 15642013**

(43) Date of publication of application:
**11.03.2015   Bulletin 2015/11**

(73) Proprietor: **Sensirion AG**
**8712 Stäfa (CH)**

(72) Inventors:
• **Wüest, Andrea**
  **CH-8712 Stäfa (CH)**
• **Gerner, Pascal**
  **CH-8712 Stäfa (CH)**
• **Böni, Dominic**
  **CH-8712 Stäfa (CH)**
• **Niederberger, Dominik**
  **CH-8712 Stäfa (CH)**
• **Sacchetti, Andrea**
  **CH-8712 Stäfa (CH)**
• **Wirz, Martin**
  **CH-8712 Stäfa (CH)**
• **Graf, Markus**
  **CH-8712 Stäfa (CH)**

(74) Representative: **Toleti, Martin**
**E.Blum & Co. AG**
**Vorderberg 11**
**8044 Zürich (CH)**

(56) References cited:
**EP-A1- 2 793 450      EP-A1- 2 796 841
EP-A1- 2 797 000      EP-A1- 2 808 650
EP-A1- 2 808 651      EP-A1- 2 808 652
WO-A1-2008/048599    WO-A1-2012/176217
WO-A2-2010/040090    JP-A- 2012 029 217
JP-A- 2014 011 765    US-A1- 2007 075 965
US-A1- 2010 257 490   US-A1- 2011 119 018
US-A1- 2011 273 378**

## Description

### Technical Field

The present invention relates to a method of determining whether a mobile device is located inside a user's pocket.

### Background of the Invention

Portable electronic devices for measuring specific environmental parameters such as temperature, pressure, humidity, gas concentrations in the ambient air etc. are well known. With the emergence of smart phones, however, a new class of devices has been more prevalent, which herein is referred to. These devices are characterized in that the measurement of an environmental parameter is not the sole purpose of the device and usually considered an additional or auxiliary task to a main function. The main function is typically a telecommunication function including the capability of communicating with public and private communication networks.

When an environmental sensor is thus integrated in such a general purpose portable device, its operation is subject to constraints very different from the operation of a specialist device for a measurement of the same parameter. In particular, the general purpose device may have to deliver a measurement of the environmental parameter, while positioned or handled in a way often not properly adapted or even detrimental to such a measurement. A typical example is the use of a mobile phone for the measurement of the ambient temperature, which without compensation can give different results depending on whether the phone is placed on a table, held in a hand or placed in a pocket. As during the usage these conditions can rapidly change, an environmental sensor is required to respond rapidly to the changing operational conditions.

It is known for example from the co-owned published United States patent application no. 2011/0307208 A1 that sensors respond to a sudden change with a change determined not only by the change itself but also by their own response function. The '208 application describes a dynamic compensation of this behaviour of the sensor in order to generate results of the measurements in shorter time than would be possible by simply waiting until the sensor reaches a new steady state.

Whilst accelerating the response time of an environmental sensor through a dynamic compensation process can alleviate some of the difficulties faced when operating such a sensor in a portable device, it is found that changes in the operating conditions are often too fast for dynamic compensation alone, which can become unstable or too slow.

Therefore it is seen as an object of the invention to further improve the operation of environmental sensors in portable electronic devices, particularly in general purpose electronic devices.

Further background art includes US 2007/075965 A1 describing various methods and devices relating to devices which, in at least certain embodiments, may include one or more sensors for providing data relating to user activity and at least one processor for causing the device to respond based on the user activity which was determined, at least in part, through the sensors. The response by the device may include a change of state of the device, and the response may be automatically performed after the user activity is determined.

WO 2008/048599 A1 discloses a control system for a mobile device comprising at least one touch sensor that generates a resistance signal based on a sensed resistance. A comparing module generates a first signal when the resistance signal is less than a threshold. A timing module determines a duration of the first signal. A selecting module communicates with the timing module and selects one of an active mode and an inactive mode of the mobile device when the duration is greater than a first period.

US 2011/0273378 A1 relates to electronic devices such as mobile devices, and methods of operating such devices. In one embodiment, the electronic device includes first and second temperature sensors positioned at different respective locations on the electronic device and at least one processing device. The first and second temperature sensors respectively output first and second signals indicative of the respective temperatures experienced at those respective sensors. The at least one processing device (i) receives the first and second signals respectively from the first and second temperature sensors respectively and generates based thereon an indication of a difference or a relationship between the first and second temperatures, and (ii) determines an operational context of the electronic device based at least in part upon the difference. In an additional embodiment, other sensor information from one or more other sensors is taken into account in determining the operational context.

### Summary of the Invention

In accordance with an aspect not claimed in the invention there is provided a portable electronic device with one or more integrated environmental sensors for measuring an environmental or ambient parameter, a compensator generating a time sequence of values used as input to the compensator in a following time step for reducing the difference between the environmental sensor output and the actual value of the environmental parameter, and a context evaluator for determining a context of the device and a re-initializer reinitializing the compensator by changing the input from the calculated value to a value depending on a context of the device as determined by the context evaluator.

The value depending on the context can be a value as calculated at a time prior to a change in context or it can be an adapted value or a newly generated value. Independent of the type of change, the changed input is derived in accordance with the result of the evaluation of the context evaluator.

The re-initialization can be seen changing an input value for the compensator from a value as calculated in the normal operation of the compensator to a value as adapted or changed depending on a context of the device as determined by the context evaluator.

As the input for re-initialization when selected correctly reflects better the changed conditions, in which the device is found, the changed value of the environmental parameter can be displayed faster than using the compensator with its normal unchanged input value.

The environmental sensor can be a temperature, a humidity, a chemical, gas, or pressure sensor. It is generally designed to measure an ambient parameter relating to a condition exterior of the housing of the device. Most of these sensors require an opening or duct in the housing to allow for the exchange of air or other fluids between the exterior and the sensing elements of the sensor. The opening can be covered with a proactive mesh, grill or membrane.

The compensator can include a dynamic compensator used to apply a correction based on a model of the response function of the sensor during a change in the environmental parameter in order to accelerate the read-out during such a change or a heat compensator used to apply a correction based on a model of the thermal propagation from heat sources in order to compensate for internal and external heat sources. More preferably the compensator operates under normal operating conditions recursively using state vectors generated at each time step as input for the next time step.

The changes in the environment and/or handling are generally caused by a corresponding change in the external or environmental conditions, also referred to as context conditions, under which the device is operated. Such changes can include a change of location such as a change from indoors (car, building) to outdoors (street), or between two locations with different ambient conditions (such as cellar vs. bathroom) or for example the exposure of the device to sun light, wind and other changing ambient conditions. The change can also be caused by the way the device is handled by a user such as a change from being held to being placed in a pocket or bag.

The change in the context condition is detected by the context evaluator, which typically resides as a programmed routine in part of general purpose computing units. However it can also include either parts of sensor processing units and via a communication links computing units at remote locations. The evaluator can be programmed to a varying degree of complexity or scenarios reflecting the diverse ways in which a portable device is typically used. The complexity can range from simple binary decisions based for example on whether a device is located within a building or outside (which in turn can be derived from a position measurement) to more complex model-based, or statistical evaluation of motion sensors, use pattern and the like to determine whether the device has been placed inside a pocket or bag. Other scenarios can be based on the current physical location (latitude, longitude) and/or semantic location (at home, at workplace) or depend for example on the current use (in communication, or play or video/music replay mode) of the device.

The context evaluator is therefore best linked to the output of sensors integrated into the device. These sensors can include the environmental sensor itself. It can however also include at least one further sensor integrated into the device. However, the change in environmental conditions can in principle also be detected by sensors external to the device and communicated to the device, such as prevalent wind strength and directions, cloud coverage and the like.

The further sensors within the meaning of the present invention can be selected from a group including picture or video cameras, IR sensors, acoustic microphones, location sensors and providers (GPS, Location fingerprinting (WiFi, GSM, network-based)), brightness sensors, ultrasound sensors, proximity sensors, acceleration sensors, Bluetooth receivers, EM wave antennae and orientation sensors, whereby these sensors are preferably integrated into the same housing as the environmental sensor or, alternatively or in addition, sensors which can communicate directly or indirectly, i.e. over a common server, with the portable device, such as external sensors in close proximity, in other mobile devices, consumer electronics and appliances, network connected sources such as internet based weather information, server based information or, alternatively or in addition, be indicators of the state of the device (stand-by, calling, gaming). Sensors to detect the handling can include a touch sensitive surface or screen of the portable device.

The portable electronic device can preferably be a mobile phone, a handheld computer, an electronic reader, a tablet computer, a game controller, a pointing device, a photo or a video camera, a digital music player, a wrist watch, a key fob, a head set, a digital photo frame and a computer peripheral, glasses, a wristband.

Other advantageous embodiments are listed in the dependent claims as well as in the description below. The described embodiments similarly pertain to the device, the method, and any computer program elements. Synergetic effects may arise from different combinations of the embodiments although they might not be described in detail. A claimed aspect of the present invention includes a method of determining a context representing the mobile device being in a pocket.

Further all embodiments of the present invention concerning a method might be carried out in the order of the steps as described. Nevertheless this has not to be the only essential order of steps but all different orders of the method steps where technically feasible shall be comprised in the scope of the claims and be disclosed by the method claims.

## Brief Description of the Drawings

The detailed description refers to examples of the present invention making reference to the annexed drawings, wherein:

FIG. 1A is a perspective view of a portable electronic device;
FIG. 1B is a schematic view into part of the housing of the device of Fig. 1A;
FIG. 2 is a block diagram with components of a portable electronic device;
FIG. 3 is a block diagram illustrating an example of the invention; and
FIGs. 4A and 4B are examples illustrating the switch between compensation with normal initialization and context-dependent re-initialization.

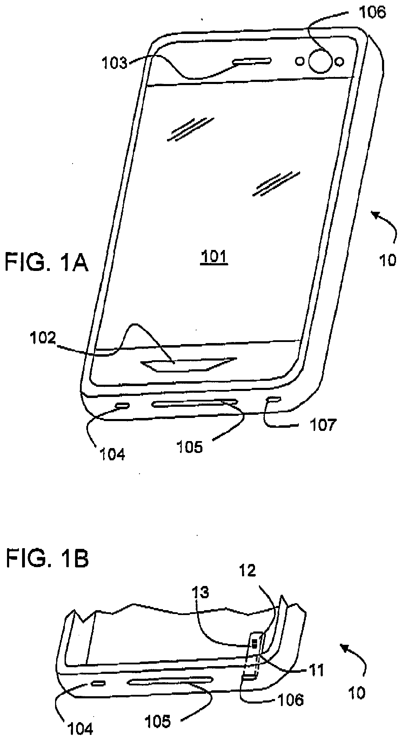

FIGs. 1A, 1B, 2 and 3 concern an aspect of the present invention which is not claimed presently.

## Detailed Description

The device of FIG. 1A (currently not claimed) is a portable electronic device such as a mobile phone. A housing 10 of the mobile phone includes a front side with a screen 101 and elements like buttons 102 to let a user interact with the phone. Also shown on the front side is an opening 103 for a loudspeaker. Further openings 104, 105 are located at a lower side wall of the housing 10. It is well known to mount components like microphones and loudspeakers behind such openings. The phone includes one or two cameras 106, and internally additional sensors (not shown) such as location sensors or GPS, or acceleration or orientation sensors in a manner well known.

Another opening 107 is located at the lower side wall. As shown in FIG. 1B (currently not claimed) the opening 107 is linked to a tubular duct 11 passing through the interior of the housing. A temperature sensor 12 and a humidity sensor 13 are both mounted along the duct 11 such that the sensitive areas of both sensors are exposed to the ambient air through the opening 107. Suitable sensors are commercially available for example from Sensirion™ AG under the tradenames SHTC1 or STS21 (as temperature only sensor).

In addition to these sensors or alternatively other environmental sensors can be used such as a gas sensors as for example proposed in: M. Afridi et. al. "MEMS-based embedded sensor virtual components for system-on-a-chip (SoC)" Solid-State Electronics 48 (2004) 1777-1781, and can be mounted within the housing 10. The actual size and shape of the duct 11 depends on the volume available and the nature of the temperature sensor 12 and the humidity sensor 13 can vary, but given the physical constraints of portable mobile devices the area of the opening is typically in the range of less than 10 square millimeters and in the present example actually about less than 3.1 square millimeters.

The temperature or other environmental sensors can also be mounted flush with the housing of the phone or even without connection to the exterior.

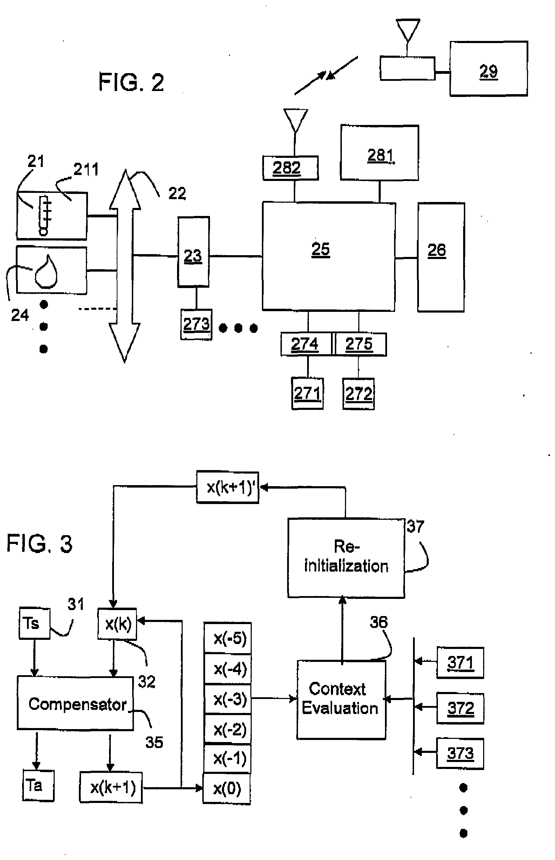

Fig. 2 (currently not claimed) shows a block diagram with the most important components of the portable device. In particular, the device includes a temperature sensor 21 integrated as part of a CMOS substrate 211 which has CMOS circuitry to control the basic functions and the basic readout of the sensor. The CMOS circuit can include for example the driver to switch the sensor and his heater on or off as well as A/D converters and amplifiers and an I2C bus controller to exchange data on an I2C bus 22. The I2C bus connects the sensors with a sensor hub 23. A further humidity sensor 24 is also linked to the I2C bus 22. The sensor hub 23 provides a control and processing unit for more complex control and read-out functions of the temperature sensor 21 based on signals sent to or extracted from, respectively, the on-chip CMOS circuitry. The sensor hub 23 also controls other auxiliary sensors such as GPS, magnetometers, accelerometers and the like.

Further control and read-out function can also be performed by a central processing unit (CPU) 25 of the portable device, which in turn has read/write access to a memory 26, which can include static or volatile memory or both as known in the art. The memory 26 typically stores the operating system of the device and can also be used to store application programs specific to the operation of the sensors of the portable device. The functions performed by the sensor hub 23 and the sensor specific programs and program libraries as stored and executed by the CPU 25 form a temperature processing unit capable of transforming the measurements of the sensor into a result which can be displayed or otherwise communicated to the user of the portable device.

The components and executable code required to perform a dynamic compensation as described for example in the above cited '208 application can reside in the memory 26 and be executed by the CPU 25.

The memory 26 and the CPU 25 can also be used to store and run executable code for a heat compensator applied to the sensor signals to correct the temperature as directly measured to compensate for effects of the surrounding of the sensor inside the portable device or external to it.

Such a compensator includes typically a representation of a model which takes into account heat sources, heat capacities and heat conduction of elements inside the device, its housing and other factors. Based on this model and measurements relating to a present status of the elements, the measured temperature value is corrected before being displayed.

In the present example the CPU 25 and the memory 26 further include and execute a system to determine whether a change in temperature is influenced by a change in the environment or handling of the mobile device. Functions of such a system are described in more detail below while making reference to FIGs. 3 to 4.

In addition to the specific sensors as described above, the CPU is also connected to one or more sensors, for example a camera 271 or a microphone 272 also shown as the camera 106 and the microphone 104 of FIG. 1. Other sensors 273 such as location, acceleration and orientation sensors can be controlled by the sensor hub 23 as shown in the example. The sensors 271, 272 communicate with the CPU 25 using their own interface units 274, 275, respectively, which operate typically in complete independence of the temperature sensor 21.

The device includes further well known input/output units 281 such as a touch sensitive display, virtual or physical keyboards and gesture tracking devices etc. The portable device as shown has a telecommunication circuit 282 comprising an antenna, driver circuits and encoding and decoding units as are well known in the art. Using such a telecommunication circuit, the device can connect to a public voice and date network and remote locations 29 as shown.

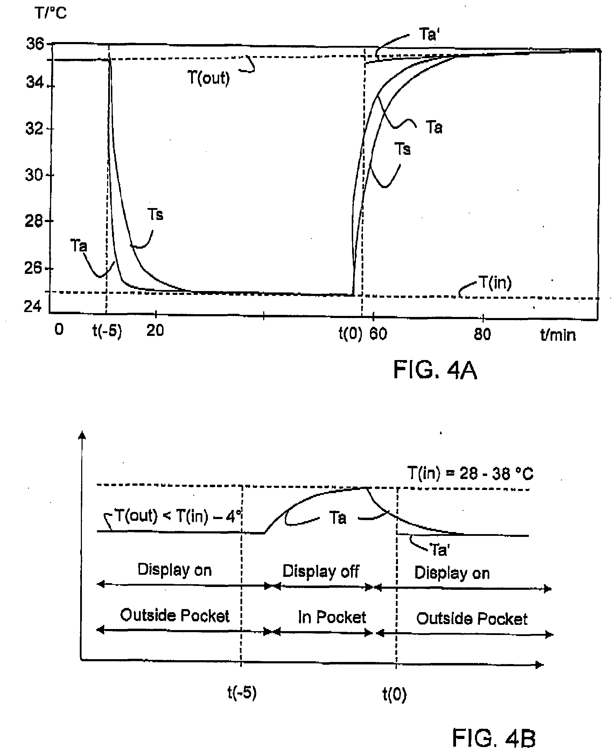

The diagrams of FIGs. 3 and 4 illustrate elements of the system used to enhance the dynamic or heat compensation through the use of contextual evaluation. Whilst realized in form of executable code in the present example, the functional elements of the context evaluation system can be implemented in other known forms of software, firmware or hardware. It should further be noted that some or all of the elements and their respective implementation can be also realized as dedicated microprocessor programmed accordingly.

The block diagram of FIG. 3 (currently not claimed) shows several sensors 371, 372, 373 as already referred to in FIG. 2. These sensors can for example be the touch sensitive screen, one or more microphones, a brightness or IR sensor, etc. In addition the temperature sensor 31 itself can provide a measurement or at least a supporting measurement used with other measurements when attempting to interpret the context in which the mobile device is used.

The compensation unit 35 as described in the following example performs a dynamic and heat compensation based on a model of the response function of the temperature sensor 31 and thermal propagation from heat sources to the sensor 31. It can be described as a program residing in the memory 26 and using the processing unit 25 of the device. The compensator can generate an estimate of the ambient temperature Ta using a suitable implementation of the following equations [1]:

$$x(k+1) = Ax(k) + Bu(k)$$

$$y(k) = Cx(k) + Du(k)$$

with u(k) denoting a representation of the reading of the temperature Ts as provided by the sensor 31 at time step $k$, y(k) denoting the output Ta, and $x(k)$ denoting an internal state vector of the compensator. $A$ is an $n$-by-$n$ matrix, $B$ an $n$-by-$m$ matrix, $C$ an $l$-by-$n$ matrix and $D$ an $l$-by-$m$ matrix, where $n$ is the number of states that depends on the complexity of the underlying model of the response function and $m$ the number of inputs.

At each time step the system takes as input the current sensor reading Ts or u(k) and the internal state vector generated in the previous iteration. Based on the model as represented by the matrix elements A - D the compensator generates at each time step a representation of an estimate of the ambient temperature Ta or y(k) and a new internal state vector x(k+1) which in turn is used as an input for the compensator 35 at the next time step. In effect, the compensator generates a time sequence of internal state vectors x(k+1), represented in FIG. 3 as register with (x(0)) to x(n-5) of the last five values.

In the example a context evaluator 36 monitors both the context in which the portable device is handled and this sequence of internal state vectors. Based on a relevant change in the context to be described in several examples below, the context evaluator 36 selects or flags an internal state vector effectively resetting the selected state vector to x(k+1), i.e., the input to the compensator.

When the context evaluator 36 later determines that the relevant change to the portable device has been reversed, a re-initializator 37 selects a state vector or recalculates a state vector based on the selected state vector and input signal (371, 372 and 373). The changed state vector is used as input vector x(k+1) (replacing the actual vector as generated by the compensator 35 in the time step) in the following time step. This operation can be regarded effectively as a re-initialization of the compensator 36 with the state vector as determined during the normal operation of the compensator replaced by a changed state vector. This operation of the context evaluator 36 and the re-initializator 37 and the resulting acceleration of the dynamic compensation is described in the following using several examples.

The context evaluator can be based on the past and/or current behaviour of the environmental sensor it controls. By tracking the changes and applying threshold or gradient tests, the context evaluator can select a state vector after registering a significant or steep change in the reading of the environmental sensor. The state vector selected form the stored state vectors can for example be a state vector prior to the occurrence of the change. When the context evaluator registers the onset of a change in the reverse direction the selected state vector can be used in the compensation as described above.

This self-referential control of the environmental sensor can work for changes between indoors and outdoor and the change in for example temperature, humidity, and air composition which can go along with stepping from an indoor location onto a street or into or out of an automobile.

In FIG. 4A there is shown an example on the operation of a device assuming a temperature difference between an outdoor temperature T(out) of 35°C slowly rising to 36°C as shown and an indoor temperature T(in) of 25°C. Due to its response function the readings of the temperature sensor follows the curve Ts. The compensator reshapes the signal of the temperature sensor to accelerate the reading resulting in a displayed temperature which follows the curve Ta.

However in case that the context evaluator registers the sudden change in temperature, which in the example is assumed to have occurred around a time step t(-5) and flags or selects the state vector at this time step, this value can be reapplied at time t(0), when the reverse step occurs and is again registered by the context evaluator. Using this modified initialization or reset of the compensator the displayed temperature follows curve Ta' which effectively re-sets the compensator with a state vector representing an ambient temperature of 35°C (as before the change to the indoor temperature). As a result the re-set compensation Ta' converges faster towards the real outdoor temperature T(out) of 36° than the normal compensation Ta.

In addition or instead of a self-referential operation the context evaluator 36 can receive inputs from other sensors 371, 372, 373 or other components of the portable device, for example CPU usage or telecommunication activity. Taking for example the change from indoor and outdoor, a GPS or a network enhanced location signal can be used by a suitable indoor/outdoor test of the context evaluator to anticipate a change in the environmental parameter measured and trigger the selection of the state vector valid prior to the change in location. When the location sensor triggers a return to initial location the compensator 35 is reset to the selected state vector.

In further examples the context evaluator can test conditions based on a multitude of sensor readings and activity indicator. To detect for example whether a mobile phone is placed in a pocket or a bag, a combination of proximity sensors, motion sensors, CPU activity, brightness detection, temperature and touch input can be combined in a statistical model predicting whether or not this scenario is true.

To detect for example whether the device is located in a pocket, the proximity sensor can be used to check on a barrier in the immediate proximity of the phone, the light or brightness sensor can be checked for reduced brightness or darkness, the display can be tested for being switched off and a temperature sensor reading can be tested for resulting in a reading close to the surface temperature of the skin (28°C -40°C). The upper limit of the temperature takes heating from other (internal and external) sources into account. More specifically a context evaluation as to whether a device is inside or outside a user's pocket can include the following conditions:

If during the period of 10 s the following conditions are true:

- Proximity Sensor indicating a close proximity, i.e. a distance value below a threshold;
- Light Sensor indicating light below a darkness threshold.

These basic conditions can be additionally accompanied by a further test to render the pocket recognition more robust. Such further test can include a temperature measurement or a test on whether the display is switched off. The temperature measurement can use the uncompensated temperature as measured by a temperature sensor in the device and a test on whether this raw temperature is between 28°C and 40°C and whether it is rising or at least stable. If these conditions are found to hold, the device is assumed to be in an "in-pocket" mode and the context evaluator initiates the adaptations to the compensator as associated with such a mode.

It should however be understood that such a context test is a probability test and not deterministic as one can never expect to recognize the true context of the device under all circumstances unless making use of direct user feedback in form of for example a direct user selection of a context.

As shown in FIG. 4B, once the context evaluator 36 has performed the above tests to determine that the in-pocket mode has started, a start value can be selected representing the state vector or initial condition of the compensator for the environmental sensor at a time t(-5) when the portable device was still in the open. When the temperature reading Ta falls to a temperature between the temperature reading at t(-5) and the maximum temperature in the in-pocket state and the context evaluator 36 determines that the device is no longer in the pocket, the initial conditions at time t(-5) are then applied at t(0) to accelerate the correct temperature display Ta'. As in the previous example the time step notation of -5 and 0 are completely arbitrary.

It should be noted that for the working of the above example, only those of the state vector or initial conditions selected by the context evaluator need to be stored.

It should be further noted that the state vector or initial condition to reset the compensator can be derived synthetically. i.e., without reference to previous values of the state vector or initial condition as used by the compensator or by using an adapted or modified version of the stored value. Given for example that the outdoor temperature at a given location is approximately known or can be extracted from remote sources such as online providers, the context evaluator can be programmed to request such information and together with the re-initializator generate a corresponding state vector or initial condition for the compensator.

While there are shown and described presently preferred embodiments of the invention, it is to be understood that the invention is not limited thereto but may be otherwise variously embodied and practised within the scope of the following claims.

**Claims**

1. A method of determining whether a mobile device is located inside a user's pocket including using a context evaluator (36) connected to sensors and/or components inside the device and testing conditions whether for a prescribed duration a proximity sensor (371,372,373) indicates closeness by a distance value below a threshold and a brightness sensor indicates a value below a darkness threshold, wherein the test includes a further condition relating to the state of a display of the device.

2. The method of claim 1, wherein the test includes at least one further condition relating to a temperature or temperature range or temperature gradient as measured by an internal temperature sensor (12,21,31).

**Patentansprüche**

1. Ein Verfahren zum Bestimmen, ob sich eine mobile Vorrichtung in der Tasche eines Benutzers befindet, einschliesslich der Verwendung eines Kontextauswerters (36), der mit Sensoren und/oder Komponenten innerhalb der Vorrichtung verbunden ist, und des Testens von Bedingungen, ob für eine vorgeschriebene Dauer ein Näherungssensor (371, 372, 373) Nähe durch einen Abstandswert unter einem Schwellenwert und ein Helligkeitssensor einen Wert unter einem Dunkelschwellenwert anzeigt, wobei der Test eine weitere Bedingung bezüglich des Zustands einer Anzeige der Vorrichtung einschliesst.

2. Das Verfahren nach Anspruch 1, wobei der Test mindestens eine weitere Bedingung einschliesst, die sich auf eine Temperatur oder einen Temperaturbereich oder einen Temperaturgradienten bezieht, wie er von einem internen Temperatursensor (12, 21, 31) gemessen wird.

**Revendications**

1. Un procédé pour déterminer si un dispositif mobile est situé à l'intérieur de la poche d'un utilisateur, comprenant l'utilisation d'un évaluateur de contexte (36) connecté à des capteurs et/ou des composants à l'intérieur du dispositif et des conditions de test pour déterminer si, pendant une durée prescrite, un capteur de proximité (371, 372, 373) indique la proximité par une valeur de distance inférieure à un seuil et un capteur de luminosité indique une valeur inférieure à un seuil d'obscurité, dans lequel le test comprend une autre condition relative à l'état d'un affichage du dispositif.

2. Le procédé selon la revendication 1, dans lequel le test comprend au moins une autre condition relative à une température ou une plage de températures ou un gradient de température tel que mesuré par un capteur de température interne (12, 21, 31).

FIG. 1A

106

103

101

102

104

105

107

10

FIG. 1B

12

13

11

106

104

105

10

FIG. 2

FIG. 3

FIG. 4A

FIG. 4B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110307208 A1
- US 2007075965 A1
- WO 2008048599 A1
- US 20110273378 A1

**Non-patent literature cited in the description**

- **M. AFRIDI.** MEMS-based embedded sensor virtual components for system-on-a-chip (SoC). *Solid-State Electronics,* 2004, vol. 48, 1777-1781